# EUROPEAN PATENT APPLICATION

(11) **EP 4 574 972 A1**
(43) Date of publication of application: **25.06.2025**
(21) Application number: 23851619.9
(22) Date of filing: 29.07.2023
(51) Int. Cl.: C12N 15/09, C12N 15/11, C12N 15/85, C12N 15/79, C12N 9/22, C12N 15/63, C07K 14/79, C07K 14/47, A01K 67/027

(54) **METHOD FOR SPECIFICALLY IMPROVING LACTOFERRIN GENE EXPRESSION AND USE THEREOF**

(30) Priority: 10.08.2022 CN 202210954987
(71) Applicant: Qingdao KingAgroot Chemical Compound Co., Ltd., Qingdao, Shandong 266000 (CN)
(72) Inventor: MO, Sudong, Qingdao, Shandong 266000 (CN); LIAN, Lei, Qingdao, Shandong 266000 (CN); JIANG, Linjian, Qingdao, Shandong 266000 (CN)
(74) Representative: De Vries & Metman
(86) International application number: PCT/CN2023/110054
(87) International publication number: WO 2024/032398

(57) **Abstract**

The present invention relates to technical fields of genetic engineering and bioinformatics, and particularly relates to a method of specifically increasing lactotransferrin gene expression levels and an application thereof. The method adopts the promoter of the specific gene, which is selected from αS1 casein gene, β casein, αS2 casein, κ casein, β-lactoglobulin or α-lactalbumin genes, to drive the *LTF* gene expression, thereby leading to up regulation of the *LTF* gene expression level in mammary glands and having great application potential in increasing the lactotransferrin content in animal milk.

## Description

### Technical Field

The present invention relates to technical fields of genetic engineering and bioinformatics, and particularly relates to a method of specifically increasing lactotransferrin gene expression levels and an application thereof.

### Background Art

Generally speaking, a complete gene expression cassette in an organism contains multiple elements including a promoter, a 5' non-coding region (5' UTR), a coding region (CDS) or non-coding RNA region (non-coding RNA), a 3' non-coding region (3' UTR), a terminator, etc. Non-coding RNA can perform its biological functions at the RNA level, including rRNA, tRNA, snRNA, snoRNA, and microRNA. A CDS region comprises exons and introns. After the transcribed RNA is translated into a protein, the amino acids in different segments usually form different domains. Specific domains determine intracellular localization and functionality (e.g., nuclear localization signal, chloroplast leading peptide, mitochondrial leading peptide, DNA binding domain, transcription activation domain, enzyme catalytic center, etc.) of the protein. For non-coding RNA, different segments also have different functions. When one or several elements of a gene change, a new gene will be formed, which may generate new functions.

Lactotransferrin (LTF) is an important iron-binding glycoprotein in milk, with a relative molecular weight of 70-80 kDa, which is mainly expressed and secreted by mammary epithelial cells and widely distributed in milk of mammals, especially in colostrum. Lactotransferrin is also contained in various other tissue secretions (saliva, tear, or bile, etc.), providing the first and most important line of defense for the mucosal system which is invaded by a large number of pathogenic microorganisms. It not only participates in the transport of iron, but also has powerful biological functions including wide-spectrum anti-bacterial, anti-oxidation, anti-cancer, immune system regulatory properties and the like, which is considered as a novel anti-bacterial and anti-cancer drug as well as a food and feed additive with great potential. It is also listed as a nutrition fortifier in the national food safety standard GB 14880. At present, the common lactotransferrin on the market is mainly bovine lactotransferrin extracted from cow milk, and then added to formula milk powder, nutritious and healthy food as well as dietary supplements, immunomodulatory health care products, etc. The lactotransferrin content has become the main indicator of the quality of these related products.

### Summary of the Invention

In order to solve the above problems existing in the prior art, the present invention provides a method of specifically increasing lactotransferrin gene expression levels and an application thereof.

The present invention provides a method of specifically increasing *the LTF* gene expression level in mammary glands. The promoter of a specific gene is employed to drive the *LTF* gene expression and the specific gene is selected from αS1 casein gene, β casein, αS2 casein, κ casein, β-lactoglobulin or α-lactalbumin genes.

In one specific embodiment, the method of specifically increasing *LTF* gene expression levels in mammary glands comprises the following steps: DNA breaks are generated simultaneously at three different specific sites in the genome of an animal cell, wherein the specific sites are respectively two genomic loci where the promoter fragment of the specific gene can be cut out and a genomic locus between the coding region of the *LTF* gene and the promoter; or a genomic locus between the promoter of the specific gene and the coding region, and two genomic loci where the coding region fragment of the *LTF* gene can be cut out. A translocation editing event, in which the promoter fragment of the specific gene is inserted upstream of the coding region of the *LTF* gene or the coding region fragment of the *LTF* gene is inserted downstream of the promoter of the specific gene, is then generated by gene editing at the above loci. Eventually, a new combination of the promoter of the specific gene and the coding region of the *LTF* gene is produced, that is, the promoter of the specific gene is employed to drive the *LTF* gene expression, thereby increasing the *LTF* gene expression level in mammary glands.

The present invention also provides a method of creating a new gene in animal cells, comprising the following steps:
(1) DNA breaks are generated simultaneously at three different specific sites in the genome of an animal cell, wherein the specific sites are respectively two genomic loci where the promoter fragment of the specific gene can be cut out and a genomic locus between the coding region of the *LTF* gene and the promoter; or a genomic locus between the promoter of the specific gene and the coding region, and two genomic loci where the coding region fragment of the *LTF* gene can be cut out. A translocation editing event, in which the promoter fragment of the specific gene is inserted upstream of the coding region of the *LTF* gene or the coding region fragment of the *LTF* gene is inserted downstream of the promoter of the specific gene, is then generated by gene editing at the above loci. Eventually, a new combination of the promoter of the specific gene and the coding region of the *LTF* gene is produced to form a new gene; the specific gene is selected from αS1 casein gene, β casein, αS2 casein, κ casein, β-lactoglobulin or α-lactalbumin genes.

In one specific embodiment, the method of creating a new gene in animal cells also comprises: (2) designing primer pairs capable of specifically distinguishing the above new combination, screening out cells or tissues containing the above new gene by PCR identification, and determining the characteristic sequence of the new combination by sequencing; and

(3) cultivating the above screened cells or tissues to obtain TO-generation animals, and performing PCR identification and sequencing on animals of two or more, or three or more consecutive generations of T0 and T1 to screen out animals that stably inherit the characteristic sequence comprising the above new combination over different generations, that is, creating a new gene that is stably inheritable in the animals;
optionally, also comprising (4) testing the functionality-related biological traits or phenotypes of the new gene, determining a genotype that can bring beneficial traits, and acquiring a new functional gene that is stably inheritable.

In one specific embodiment, the "three different specific sites" may be located on the same chromosome or on different chromosomes.

In one specific embodiment, the "three different specific sites" may be at specific sites on three different genes, or at three different specific sites on the same gene.

In one specific embodiment, the transcription directions of the "three different genes" may be the same or different.

In one specific embodiment, the "DNA break" is achieved by delivering a nuclease with targeting properties into a cell of an organism to contact with specific sites of the genomic DNA. Such DNA breaks have no substantial difference from DNA breaks created by traditional technical means (e.g., radiation or chemical mutagenesis).

In one specific embodiment, the "nuclease with targeting properties" includes meganuclease, zinc finger nuclease (ZFN), TALEN, and CRISPR/Cas systems,
wherein, the CRISPR/Cas system can simultaneously create three DNA double-strand breaks at different sites of the genome through three guide RNAs that target different sequences; the zinc finger nuclease and TALEN systems can simultaneously create three DNA double-strand breaks by respectively designing ZFN proteins or TALEN proteins for sequences of the three specific sites.

In one specific embodiment, the CRISPR/Cas system is a Cas9 nuclease system or a Cas12 nuclease system.

In one specific embodiment, the "nuclease with targeting properties" exists in the form of DNA.

In another specific embodiment, the "nuclease with targeting properties" exists in the form of mRNA or a protein instead of DNA.

In one specific embodiment, the method of delivering a nuclease with targeting properties into a cell is selected from 1) PEG-mediated cell transfection method; 2) liposome-mediated cell transfection method; 3) electroporation transformation method; 4) microinjection; 5) particle bombardment; 6) Agrobacterium-mediated transformation method; 7) viral vector-mediated transformation method; or 8) nano-magnetic bead transformation method.

The present invention further provides a new gene produced by the method of creating a new gene in animal cells.

The present invention further provides an application of the gene in increasing the lactotransferrin content in animal milk (preferably cow milk).

The present invention further provides a DNA comprising the gene.

The present invention further provides a protein encoded using the gene, or a bioactive fragment thereof.

The present invention further provides a recombinant expression vector comprising the gene, and a promoter operably linked thereto.

The present invention further provides an expression cassette comprising the gene.

The present invention further provides an animal cell, wherein it comprises the expression cassette.

The present invention further provides an animal regenerated using the animal cell.

The present invention further provides a composition, which comprises the promoter of the specific gene and the coding region of the *LTF* gene, wherein, the specific gene is selected from αS1 casein gene, β casein, αS2 casein, κ casein, β-lactoglobulin or α-lactalbumin genes. The composition is non-naturally occurring and is directly linked on animal chromosomes and can be stably inherited; preferably, it fuses in vivo.

The present invention has the following excellent effects: A target site combination for gene editing is designed near the specific elements of the target gene to cause DNA double-strand breaks, and spontaneous repair and joining. Oriented combination of the promoter of the specific gene and the coding region of the *LTF* gene can be achieved at the genomic level without the need to provide an exogenous DNA template, thereby enabling the promoter of the specific gene to drive the *LTF* gene expression, leading to up regulation of the *LTF* gene expression level in mammary glands. It has great application potential in increasing the lactotransferrin content in animal milk.

### Detailed Description of the Invention

In the present invention, unless otherwise specified, scientific and technical terms used herein have the meanings as commonly understood by a person skilled in the art. Moreover, the terms and laboratory procedures related to protein and nucleic acid chemistry, molecular biology, cell and tissue culture, microbiology, immunology, etc. used herein are all terms and routine procedures widely used in corresponding fields. For example, the standard recombinant DNA and molecular cloning techniques used in the present invention are well known to a person skilled in the art, and are more fully described in Sambrook, J., Fritsch, E.F. and Maniatis, T., Molecular Cloning: A Laboratory Manual, Cold Spring Harbor Laboratory Press, Cold Spring Harbor, 1989. Meanwhile, to better understand the present invention, definitions and explanations of the related terms are provided below.

The term "genome" used herein refers to a complete complement to genetic material (genes and non-coding sequences) present in each cell or virus or organelle, and/or a complete chromosome complement inherited from a parent as a unit (haploid) in an organism.

The "up regulation of gene expression levels" of the present invention refers to an increased expression level of the new gene relative to the endogenous wild-type gene of the corresponding organism, preferably the expression level is increased by at least 0.5-fold, at least 1-fold, at least 2-fold, at least 3-fold, at least 4-fold, or at least 5-fold.

The term "gene editing" refers to a strategy and technique for targeting-specific modifications of any genetic information or genome of a living organism. Thus, gene editing not only includes editing of gene coding regions, but also includes editing of regions other than the gene coding regions of a genome. It also includes editing or modifying the nucleus (if present) and other genetic information of a cell.

The term "transgene" is used herein to describe genetic material that has been or is to be artificially introduced into the genome of a host organism and is to be passed on to progeny of the host. A transgene will generally comprise polynucleotides which comprise non-coding and/or coding sequences that usually, but not necessarily, affect or cause activity (e.g., regulation of transcription or translation, generation of nucleotide sequences comprising coding and/or non-coding sequences, etc.).

The term "CRISPR/Cas nuclease" may be a CRISPR-based nuclease or a nucleic acid sequence encoding it, including but not limited to: 1) Cas9, including SpCas9, ScCas9, SaCas9, xCas9, VRER-Cas9, EQR-Cas9, SpG-Cas9, SpRY-Cas9, SpCas9-NG, NG-Cas9, NGA-Cas9 (VQR), etc.; 2) Cas12, including LnCpf1, LbCpf1, FnCpf1, AsCpf1, MAD7, etc., or any variant or derivative of the aforementioned CRISPR-based nucleases, preferably, wherein at least one of the CRISPR-based nucleases comprises a mutation compared with the corresponding wild-type sequence, such that the obtained CRISPR-based nuclease recognizes a different PAM sequence. As used herein, the "CRISPR-based nuclease" is any nuclease that has been identified in a naturally occurring CRISPR system, which has subsequently been isolated from its natural background and has preferably been modified or combined into a recombinant construct of interest, suitable as a tool for targeted genome engineering. As long as the original wild-type CRISPR-based nuclease provides DNA recognition, i.e., binding properties, any CRISPR-based nuclease may be employed and optionally reprogrammed or otherwise mutated to suit various embodiments of the present invention.

The term "CRISPR" refers to a sequence-specific genetic manipulation technique dependent on the Clustered Regularly Interspaced Short Palindromic Repeats approach, differing from RNA interference that regulates gene expression at the transcriptional level.

The "Cas9 nuclease" and "Cas9" are used interchangeably herein, referring to an RNA-guided nuclease comprising a Cas9 protein or a fragment thereof (e.g., including a protein comprising an active DNA cleavage domain of Cas9 and/or gRNA-binding domain of Cas9). Cas9 is a component of the CRISPR/Cas (Clustered Regularly Interspaced Short Palindromic Repeats and related systems thereof) gene editing system, capable of targeting and cutting DNA target sequences under the guidance of guide RNA to form DNA double-strand breaks (DSB).

The "Cas protein" or "Cas polypeptide" refers to a polypeptide encoded by a Cas (CRISPR-related) gene. A Cas protein includes Cas endonuclease. A Cas protein may be a bacterial or archaeal protein. For example, the Type I-III CRISPR Cas proteins herein usually originate from prokaryotes; the Type I and Type III Cas proteins may be derived from bacterial or archaeal species, while the Type II Cas proteins (i.e., Cas9) may be derived from bacterial varieties. A Cas protein includes Cas9 protein, Cpf1 protein, C2c1 protein, C2c2 protein, C2c3 protein, Cas3, Cas3-HD, Cas5, Cas7, Cas8, Cas10, Cas12a, Cas12b, or a composition or complex of these.

The "Cas9 variant" or "Cas9 endonuclease variant" refers to a variant of the parent Cas9 endonuclease, wherein when associated with crRNA and tracRNA, or associated with sgRNA, the Cas9 endonuclease variant reserves the following abilities: recognizing and binding all or part of the DNA target sequence and optionally unwinding all or part of the DNA target sequence, nicking all or part of the DNA target sequence, or cutting all or part of the DNA target sequence. A Cas9 endonuclease variant includes the Cas9 endonuclease variant described herein, wherein the Cas9 endonuclease variant differs from the parent Cas9 endonuclease in such a way that the Cas9 endonuclease variant (when complexed with gRNA to form a polynucleotide-guided endonuclease complex that can modify the target site) has at least one improved property, for example, but not limited to, increased transformation efficiency, increased DNA editing efficiency, reduced off-target cutting, or any combination thereof, when compared with the parent Cas9 endonuclease (when complexed with the same 1gRNA to form a polynucleotide-guided endonuclease complex that can modify the same target site).

The Cas9 endonuclease variant described herein includes a variant that can bind to the target site of a double-stranded DNA and nick at the target site of the double-stranded DNA when associated with crRNA and tracrRNA or associated with sgRNA, while the parent Cas endonuclease can bind to the target site and break (cut) the double strands when associated with crRNA and tracrRNA or associated with sgRNA.

The "guide RNA" and "gRNA" are used interchangeably herein, referring to a guide RNA sequence which is corrected using CRISPR techniques in order to target a specific gene, usually consisting of partially complementary crRNA and tracrRNA molecules that form a complex, wherein the crRNA comprises a sequence with sufficient complementarity to the target sequence so as to hybridize with the target sequence and guide the CRISPR complex (Cas9 + crRNA + tracrRNA) to sequence-specifically bind to the target sequence. However, it is known in the art that single guide RNA (sgRNA) can be designed, which comprises features of both crRNA and tracrRNA.

The terms "single guide RNA" and "sgRNA" are used interchangeably herein and relate to a synthetic fusion of two RNA molecules, wherein the fusion of a crRNA (CRISPR RNA) comprising a variable targeting domain (linked to a tracr mate sequence hybridizing to a tracrRNA) and a tracrRNA (transactivating CRISPR RNA) is included. The sgRNA may include a crRNA or crRNA fragment and a tracrRNA or tracrRNA fragment of the Type II CRISPR/Cas system that can form a complex with a Type II Cas endonuclease, wherein the guide RNA/Cas endonuclease complex can guide the Cas endonuclease to a DNA target site, enabling the Cas endonuclease to recognize and optionally bind to the DNA target site, and optionally nick the DNA target site or cut (introduce a single-strand or double-strand break) the DNA target site.

In some embodiments, guide RNA (one or more) and Cas9 can be delivered to a cell as a ribonucleoprotein (RNP) complex. A RNP is composed of a purified Cas9 protein complexed with gRNA, and it is well known in the art that a RNP can be effectively delivered to cells of many types, including but not limited to stem cells and immune cells (Addgene, Cambridge, MA, Mirus Bio LLC, Madison, WI).

The protospacer adjacent motif (PAM) herein refers to a short nucleotide sequence adjacent to a target sequence (protospacer) recognized (targeted) by a gRNA/Cas endonuclease system. If a target DNA sequence is not adjacent to a suitable PAM sequence, the Cas endonuclease may not successfully recognize the target DNA sequence. The sequence and length of a PAM herein may vary depending on the Cas protein or Cas protein complex used. The PAM sequence may be of any length, but typically 1, 2, 3, 4, 5, 6, 7, 8, 9, 10, 11, 12, 13, 14, 15, 16, 17, 18, 19 or 20 nucleotides long.

The term "animal" includes individual animals at various developmental stage (including neonatal, embryonic and fetal stages), for example, humans, non-human mammals [e.g., primates (such as lemurs, tarsiers, monkeys, apes and orangutans), pigs, cattle, sheep, horses, camels, rabbits, kangaroos, deer, polar bears, canids (such as dogs, wolves, foxes and jackals), felines (such as lions, tigers, cheetahs, lynxes and cats) and rodents (such as mice, rats, hamsters and guinea pigs)], etc. The term "non-human" does not include humans.

The term "gene" includes a nucleic acid fragment expressing a functional molecule (such as, but not limited to, a specific protein), including regulatory sequences before (5' non-coding sequences) and after (3' non-coding sequences) a coding sequence.

The DNA sequence "encoding" a specific RNA is a DNA nucleic acid sequence that is transcribed into the RNA. A DNA polynucleotide can encode RNA (mRNA) that is translated into a protein, or a DNA polynucleotide can encode RNA that is not translated into a protein (e.g., tRNA, rRNA or DNA-targeting RNA; which is also known as "non-coding" RNA or "ncRNA").

The "polypeptide", "peptide", and "protein" are used interchangeably in the present invention, referring to a polymer of amino acid residues. The terms are applicable to amino acid polymers in which one or more amino acid residues are artificially chemical analogs of the corresponding naturally occurring amino acids and are applicable to naturally occurring amino acid polymers. The terms "polypeptide", "peptide", "amino acid sequence" and "protein" may also include modification forms, including but not limited to glycosylation, lipid attachment, sulfation, γ-carboxylation of glutamic acid residues, hydroxylation and ADP-ribosylation.

The "bioactive fragment" refers to a fragment that has one or more amino acid residues deleted from the N- and/or C-terminus of a protein while still retaining its functional activity.

The terms "polynucleotide" and "nucleic acid" are used interchangeably, including DNA, RNA, or a hybrid thereof, which may be double-stranded or single-stranded.

The terms "nucleotide sequence" and "nucleic acid sequence" both refer to the sequence of bases in DNA or RNA.

A person of ordinary skill in the art can easily adopt known methods such as directed evolution and point mutation methods to mutate the DNA fragments of the present invention. Those artificially modified nucleotides having at least 75% identity to a DNA fragment shown in any of the aforementioned sequences of the present invention and having the same functionality are all nucleotide sequences derived from the present invention and are equivalent to the sequences of the present invention.

The term "identity" refers to the sequence similarity to a natural nucleic acid sequence. Identity may be evaluated by observation with naked eye or by computer software. Using computer software for sequence alignment, the identity between two or more sequences can be represented by a percentage (%) which can be used for evaluating the identity between related sequences. The "partial sequence" means at least 5%, 10%, 20%, 30%, 40%, 50%, 60%, 70%, 80%, 90% or 95% of a given sequence.

The stringent conditions may be as follows: hybridizing at 50°C in a mixed solution of 7% sodium dodecyl sulfate (SDS), 0.5 M NaPO₄ and 1 mM EDTA, and rinsing at 50°C in 2×SSC and 0.1% SDS; alternatively: hybridizing at 50°C in a mixed solution of 7% SDS, 0.5 M NaPO₄ and 1 mM EDTA, and rinsing at 50°C in 1×SSC and 0.1% SDS; alternatively: hybridizing at 50°C in a mixed solution of 7% SDS, 0.5 M NaPO₄ and 1 mM EDTA, and rinsing at 50°C in 0.5×SSC and 0.1% SDS; alternatively: hybridizing at 50°C in a mixed solution of 7% SDS, 0.5 M NaPO₄ and 1 mM EDTA, and rinsing at 50°C in 0.1×SSC and 0.1% SDS; alternatively: hybridizing at 50°C in a mixed solution of 7% SDS, 0.5 M NaPO₄ and 1 mM EDTA, and rinsing at 65°C in 0.1×SSC and 0.1% SDS; alternatively: hybridizing at 65°C in a solution of 6×SSC and 0.5% SDS, and then washing the filter once with each of 2×SSC + 0.1% SDS and 1×SSC + 0.1% SDS; alternatively: in a solution of 2×SSC and 0.1% SDS, hybridizing and washing the filter twice at 68°C, 5 min each time, further in a solution of 0.5×SSC and 0.1% SDS, hybridizing and washing the filter twice at 68°C, 15 min each time; alternatively: hybridizing and washing the filter in a solution of 0.1×SSPE (or 0.1×SSC) and 0.1% SDS at 65°C.

As used in the present invention, the "expression cassette", "expression vector" and "expression construct" refer to vectors suitable for expression in plants of a nucleotide sequence of interest, for example a recombinant vector. The "expression" refers to the production of a functional product. For example, expression of a nucleotide sequence may refer to transcription of the nucleotide sequence (e.g., transcription to produce mRNA or functional RNA) and/or translation of the RNA into a precursor or mature protein.

The "expression construct" of the present invention may be a linear nucleic acid fragment, circular plasmid, viral vector, or, in some embodiments, may be translatable RNA (e.g., mRNA).

The "expression construct" of the present invention may comprise regulatory sequences from different sources and a nucleotide sequence of interest, or regulatory sequences of the same origin but arranged in a manner different from that normally found in nature and a nucleotide sequence of interest.

The "highly-expressing gene" refers to a gene whose expression level is higher than that of a normal gene in specific tissue.

The terms "recombinant expression vector" or "DNA construct" are used interchangeably herein, referring to a DNA molecule comprising a vector and at least one insert. A recombinant expression vector is produced for the purpose of expressing and/or reproducing the insert, or for the purpose of constructing other recombinant nucleotide sequences. An insert may be operably or may not be operably linked to a promoter sequence and may be operably or may not be operably linked to a DNA regulatory sequence.

The "regulatory sequence" and "regulatory element" are used interchangeably, referring to a nucleotide sequence located upstream (5' non-coding sequence), in the middle, or downstream (3' non-coding sequence) of a coding sequence, and affecting the transcription, RNA processing or stability, or translation of the related coding sequence. A plant expression regulatory element refers to a nucleotide sequence capable of controlling the transcription, RNA processing or stability, or translation of a nucleotide sequence of interest in plants.

A regulatory sequence may include, but is not limited to, a promoter, translation leader sequence, intron, and polyA recognition sequence.

The "promoter" refers to a nucleic acid fragment capable of controlling the transcription of another nucleic acid fragment. In some embodiments of the present invention, the promoter is a promoter capable of controlling gene transcription in plant cells, regardless of whether it is derived from a plant cell. A promoter may be a constitutive promoter or a tissue-specific promoter or a developmentally regulated promoter or an inducible promoter.

The term "strong promoter" is a well-known and widely used term in the art. Many strong promoters are known in the art or can be identified by routine experiments. The activity of the promoter is higher than the activity of the promoter effectively linked to the nucleic acid molecule to be overexpressed in a wild-type organism, for example, a promoter whose activity is higher than that of the promoter of an endogenous gene. Preferably, the activity of a strong promoter is higher by about 2%, 5%, 8%, 10%, 20%, 30%, 40%, 50%, 60%, 70%, 80%, 90%, 100%, 150%, 200%, 250%, 300%, 350%, 400%, 450%, 500%, 600%, 700%, 800%, 900%, 1000% or more than 1000% than the activity of the promoter effectively linked to the nucleic acid molecule to be overexpressed in a wild-type organism. A person skilled in the art knows how to measure the activity of a promoter and compare activity of different promoters.

The "constitutive promoter" refers to a promoter that will generally cause a gene to be expressed in most cell types under most circumstances. The "tissue-specific promoter" and "tissue-preferred promoter" are used interchangeably, and refer to a promoter that is mainly, but not necessarily exclusively, expressed in a type of tissue or an organ, and may also be expressed in a specific cell or cell type. The "developmentally regulated promoter" refers to a promoter whose activity is determined by a developmental event. The "inducible promoter" responds to an endogenous or exogenous stimulus (environment, hormone, chemical signal, etc.) to selectively express an operably linked DNA sequence.

As used herein, the term "operably link" refers to linking a regulatory element (such as, but not limited to, promoter sequence, transcription termination sequence, etc.) to a nucleic acid sequence (e.g., coding sequence or open reading frame) such that the transcription of the nucleotide sequence is controlled and regulated by the transcription regulatory element. The techniques used for operably linking a regulatory element region to a nucleic acid molecule are known in the art.

"Introducing" a nucleic acid molecule (e.g., plasmid, linear nucleic acid fragment, RNA, etc.) or protein into a plant refers to transforming a plant cell with the nucleic acid or protein, enabling the nucleic acid or protein to function in the plant cell. The "transformation" used in the present invention includes stable transformation and transient transformation.

The "stable transformation" refers to that the introduction of an exogenous nucleotide sequence into a plant genome leads to a stable inheritance of the exogenous gene. Once stably transformed, the exogenous nucleic acid sequence is stably integrated into the genome of the plant and any successive generations thereof.

The "transient transformation" refers to that the introduction of a nucleic acid molecule or protein into a plant cell to perform functions does not lead to a stable inheritance of the exogenous gene. In transient transformation, the exogenous nucleic acid sequence is not integrated into the plant genome.

Unless defined otherwise, all technical terms and scientific terms used herein have the same meanings as commonly understood by one of ordinary skill in the art to which the present invention pertains. Although any methods and materials similar or equivalent to those described herein may also be used in the practice or testing of the present invention, the preferred methods and materials are described hereby.

All publications and patents cited in this specification are incorporated herein by reference as if each individual publication or patent was incorporated by reference by means of being specifically and individually indicated and are incorporated herein by reference to disclose and describe methods and/or materials related to the publications cited. The citation of any publication, which is published prior to its filing date, should not be construed as an admission that the present invention is not eligible to precede the publication of the prior invention. In addition, the publication date provided may differ from the actual publication date which may require independent verification.

Unless specifically stated or implied, as used herein, the terms "a", "a/an", and "the" mean "at least one". All patents, patent applications, and publications mentioned or cited herein are incorporated herein by reference in their entirety, with the same degree of citation as if they were individually cited.

### Description of Figures

Figure 1 shows the schematic of the *CSN1S1* gene promoter translocated upstream of the *LTF* gene in cattle;
Figure 2 shows the schematic of the *LTF* gene translocated downstream of *the CSN1S1* gene promoter in cattle.
Figure 3 shows the schematic of promoters of different lengths for different genes.
Figure 4 shows the schematic of ligation of promoters of different lengths for the different genes and the bovine *LTF* coding sequence.
Figure 5 shows the plasmids used for transgenosis.
Figure 6 shows the mRNAassay of lactotransferrin in the transgenic bovine epithelial cells.
Figure 7 shows the ELISA assay of lactotransferrin protein content in the supernatant of the transgenic bovine epithelial cells.
Figure 8 shows the lactotransferrin expression levels in the milk of the transgenic mice.
Figure 9 shows the sequence maps of the junctions between the gene-edited *CSN1S1, CSN2* promoters and the *LTF* gene.
Figure 10 shows the identification of lactotransferrin expression of mammary epithelial cells knocked up by CRISPR/Cas9 gene editing.
Figure 11 shows the identification of lactotransferrin expression of mammary epithelial cells knocked up by CRISPR/Cas12 gene editing.

### Detailed Embodiments of the Invention

The present invention is further illustrated below with reference to examples. The following description is by way of example, but the claimed scope of the present invention should not be limited thereto.

### Example 1 Gene knock up of lactotransferrin gene expression in mammary epithelial cells with the aid of the promoters of other genes

In view of the high protein content of β casein (CSN2, casein beta, NCBI Gene ID: 281099), αS1 casein (CSN1S1, casein alpha-S1, NCBI Gene ID: 282208), αS2 casein (CSN1S2, casein alpha-S2 , NCBI Gene ID: 282209), κ casein (CSN3, casein kappa, NCBI Gene ID: 281728) , β-lactoglobulin (PAEP, beta-lactoglobulin, NCBI Gene ID: 280838), α-lactalbumin genes (LALBA, alpha-lactalbumin, Gene ID: 281894), etc. in cow milk protein, we obtained the mRNA expression levels of the *CSN1S1, CSN1S2, CSN2, CSN3, PAEP* and *LALBA* transcripts in mammary epithelial cells of lactating cows from the NCBI database, and the results showed that their expression levels were all higher than that of the *LTF* (NCBI Gene ID: 280846), therefore the promoter fragments of different lengths for the above genes were employed to knock up the lactotransferrin expression level in the present invention.

### Example 2 Testing the effects of the promoters of different genes driving the bovine lactotransferrin gene to increase the lactotransferrin content in bovine mammary epithelial cells

The promoters of the following genes, β casein (CSN2, casein beta, NCBI Gene ID: 281099), αS1 casein (CSN1S1, casein alpha-S1, NCBI Gene ID: 282208), αS2 casein (CSN1S2, casein alpha-S2, NCBI Gene ID: 282209), κ casein (CSN3, casein kappa, NCBI Gene ID: 281728), β-lactoglobulin (PAEP, beta-lactoglobulin; NCBI Gene ID: 280838), α-lactalbumin genes (LALBA, alpha-lactalbumin; Gene ID: 281894), were selected to drive the bovine lactotransferrin gene and construct related vectors to transform bovine mammary epithelial cells. Analysis of the lactotransferrin gene expression level and determination of the protein content thereof were carried out for the transgenic bovine mammary epithelial cells.

### 1. Primary culture of dairy cattle mammary epithelial cells

The mammary gland tissue of the lactating cloned Holstein dairy cattle was aseptically collected, and the middle part of the breast was sterilized according to conventional operations. A small amount of mammary gland tissue was cut out, isolated and trimmed on an ultra-clean workbench, washed with D-Hanks solution for 3-5 times until it was clean. The adipose tissue and connective tissue were removed as much as possible, and the white granular acinar tissue was cut into pieces as much as possible. The tissue was about 1 mm³, which was inoculated in a culture flask with 0.5% active collagen at a proper density using a pipette, added with 3 mL of 15% DF12 complete culture solution, cultured at 37°C in an incubator with 5% CO₂.

### 2. Passage culture and purification of dairy cattle mammary epithelial cells

When the primary cells were cultured to day 3, the media were replaced with fresh ones for the cells, and thereafter the media were replaced every other day. On the 6^{th} day or so of culture, a small number of fibroblasts began to appear around the tissue. On the 10^{th} day or so, mammary epithelial cells began to grow around the tissue. When a single layer of cells were confluent to about 90% of the bottom of the flask, the original culture solution was aspirated. According to the different sensitivities of epithelial cells and fibroblasts to trypsin digestion, the mixed-growing primary or passage cell cultures were digested by 0.25% trypsin in D-Hanks solution at 37°C, and then the cells were observed under an inverted microscope. Until the fibroblast cytoplasm retracted, intercellular space increased, and the cells detached from the wall of the flask, culture solution was added to terminate the digestion. The enzyme solution was poured out, and most of the products obtained after centrifugation were fibroblasts. After selection and passages of 3-5 times, the tissue was discarded, and purified mammary epithelial cells can be obtained.

### 3. Preparation of transgenic plasmids

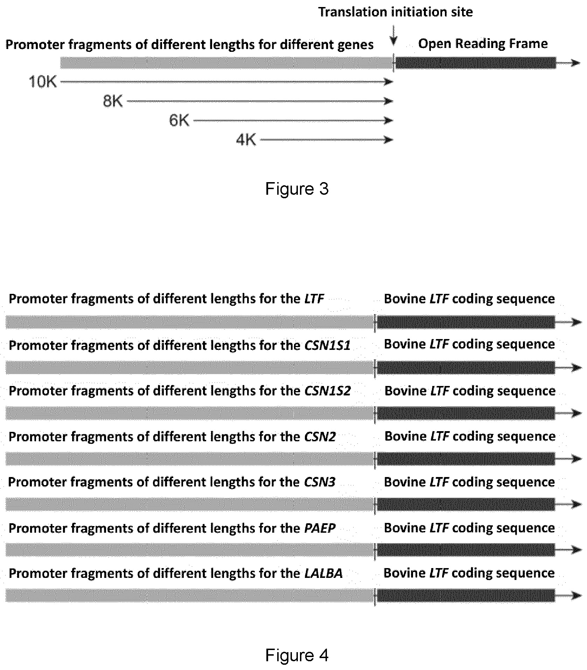

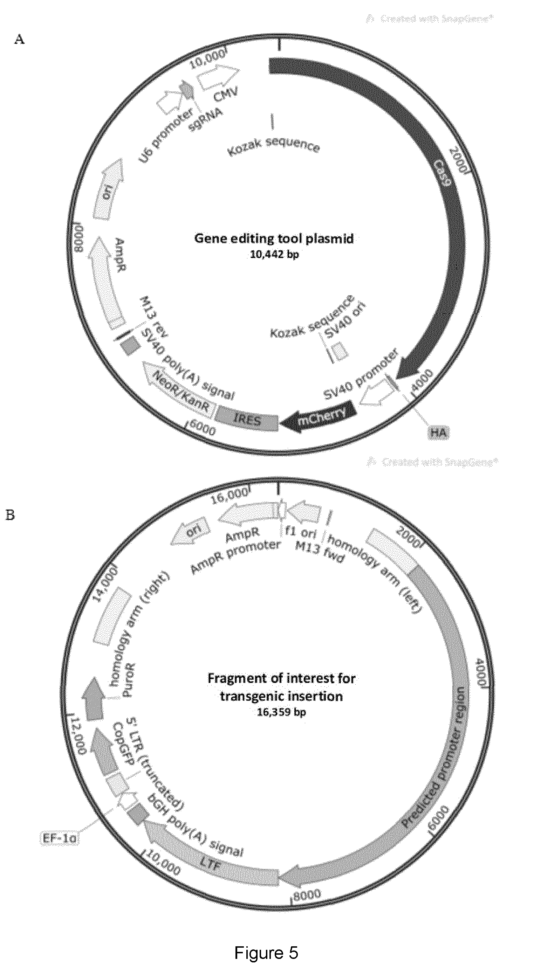

The fragments 4K, 6K, 8K and 10K ahead of the translation initiation sites of the bovine *LTF, CSN1S1, CSN1S2, CSN2, CSN3, PAEP* and *LALBA* genes (Figure 3) were respectively obtained, individually ligated with the bovine *LTF* coding sequence (Figure 4), connected into the plasmid with the drug selection marker (Figure 5-B), and used together with the CRISPR/Cas9 gene editing tool plasmid (Figure 5-A) to insert the fragment of interest between the *LTF* promoter and the open reading frame in the bovine genome in order to block the expression of endogenous lactotransferrin.

### 4. Transfection of transgenic plasmids and assay of lactotransferrin expression

The above-mentioned constructed plasmids were individually transfected into the constructed mammary epithelial cells during lactation. The successfully transfected cells were screened by drugs and flow cytometry 48 hours after transfection. After 2 passages, the cells and cell supernatant were collected separately, and mRNA assay of lactotransferrin in the cells and ELISA assay of the lactotransferrin protein content in the supernatant were performed.

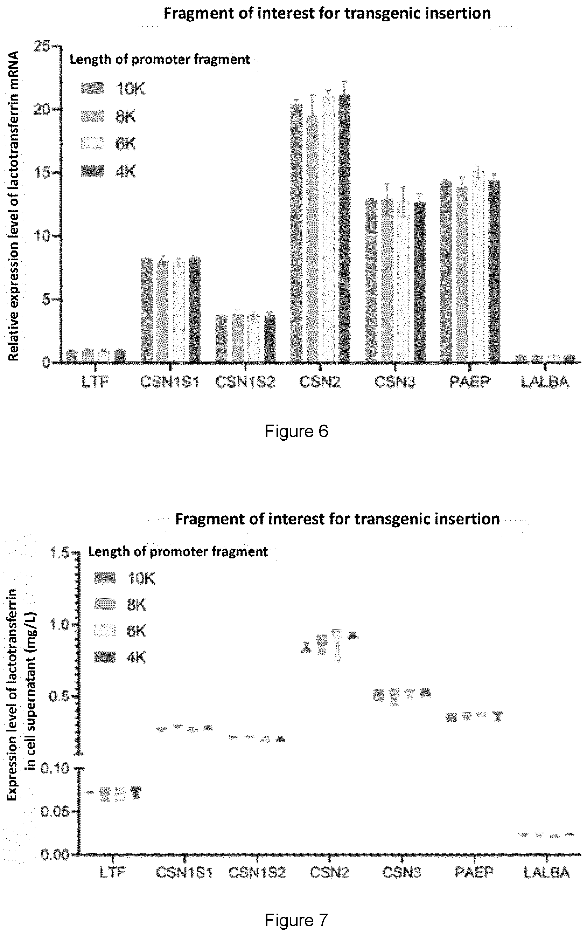

As shown in Figure 6 and 7, the assay results showed that, compared with its own promoter of the bovine *LTF,* the *CSN1S1, CSN1S2, CSN2, CSN3* and *PAEP* promoters could significantly increase the mRNA and protein expression levels of the *LTF,* among which the *CSN2* promoter had the most obvious increasing effect, followed by the promoters of the *CSN3, PAEP,* CSN1S1 and *CSN1S2* in sequence; unexpectedly, even though the expression of the *LALBA* was higher than that of the *LTF* in cow milk, the expression level of lactotransferrin promoted by the *LALBA* promoter was significantly decreased; in addition, it was worthy noting that the promoters of different lengths for the 7 genes, the *LTF,* the *CSN1S1,* the *CSN1S2,* the *CSN2,* the *CSN3,* the *PAEP* and the *LALBA,* could all effectively promote the expression of lactotransferrin .

### Example 3 Testing the effects of the promoters of different genes driving the bovine lactotransferrin gene to increase the lactotransferrin content in mice

The fragments 4K, 6K, 8K and 10K ahead of the translation initiation sites of the bovine *LTF, CSN1S1, CSN1S2, CSN2, CSN3, PAEP* and *LALBA* genes (Figure 3) were obtained, individually ligated with the bovine *LTF* coding sequence (Figure 4), and connected into the plasmid with the drug selection marker (Figure 5-B). The CRISPR/Cas9 gene editing tool plasmid was constructed (Figure 5-A). The fragment of interest was inserted between the *LTF* promoter and the open reading frame in the mice genome in order to block the expression of endogenous lactotransferrin in mice and drive the expression of the transgenic bovine lactotransferrin. The plasmid fibers were injected into the pronuclei of zygotes of the mice to obtain transgenic mice of F0 generation. The female transgenic mice of F1 generation were bred and the content of bovine lactotransferrin in the milk of the F1-generation transgenic mice was determined after pregnancy.

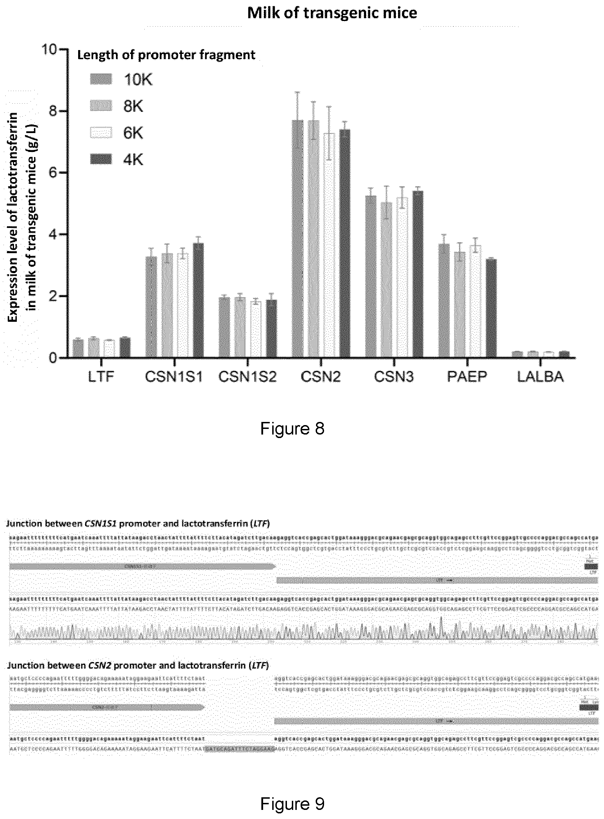

The results showed that, compared with its own promoter of the bovine *LTF,* the bovine *CSN1S1, CSN1S2, CSN2, CSN3* and *PAEP* promoters could significantly increase protein expression levels of the bovine lactotransferrin in the milk of the transgenic female mice, among which the *CSN2* promoter had the most obvious increasing effect, followed by the promoters of the *CSN3, CSN1S1, PAEP* and *CSN1S2* in sequence; while the expression level of lactotransferrin promoted by the *LALBA* promoter was significantly decreased (Figure 8).

The above results of the transgenic mice were the same as the results of the cell transfection, both indicating the promoter fragments of the bovine *CSN1S1, CSN1S2, CSN2, CSN3* and *PAEP* genes could effectively increase the expression of lactotransferrin.

### Example 4 Design of target sites for gene editing knock-up

The promoter fragments 4K ahead of the translation initiation sites of the *CSN1S1, CSN1S2, CSN2, CSN3, PAEP* and *LALBA* genes were selected in the present invention for gene editing to knock up bovine lactotransferrin.

### 1. Design of target sites of CRISPR/Cas9:

Using the online CRISPR target site design tool (crispor.tefor.net), target sites of CRISPR/Cas9 were respectively designed upstream and downstream of the bovine *LTF* gene, and target sites were respectively designed upstream and downstream of the 4K fragments of the promoters of the bovine *LTF, CSN1S1, CSN1S2, CSN2, CSN3, PAEP* and *LALBA.* The sequences of the target sites synthesized by GenScript (Nanjing) Co., Ltd. are as follows:
sgRNA1: upstream of the *LTF* gene 5' cagtgctcggtgacctcacg 3'
sgRNA2: downstream of the *LTF* gene 5' aataattagcctccgtgaat 3'
sgRNA3: upstream of the *CSN1S1* gene promoter 5' acctctactgtgtaatcata 3'
sgRNA4: downstream of the *CSN1S1* gene promoter 5' gagaagtttcatggttgtca 3'
sgRNA5: upstream of the *CSN1S2* gene promoter 5' tactggtatggcagatgttg 3'
sgRNA6: downstream of the *CSN1S2* gene promoter 5' tagcttcttgtctatctcac 3'
sgRNA7: upstream of the *CSN2* gene promoter 5' cccatggactgcagcctaac 3'
sgRNA8: downstream of the *CSN2* gene promoter 5' ttctaatcatgcagatttct 3'
sgRNA9: upstream of the *CSN3* gene promoter 5' aattgatagttgcaagatta 3'
sgRNA10: downstream of the *CSN3* gene promoter 5' tttaattttaggtgcaatga 3'
sgRNA 11: upstream of the *PAEP* gene promoter 5' aaagctacagatatagtcat 3'
sgRNA12: downstream of the *PAEP* gene promoter 5' aggcacttcatggctgcagc 3'
sgRNA13: upstream of the *LALBA* gene promoter 5' tatctcttcttgctattctt 3'
sgRNA14: downstream of the *LALBA* gene promoter 5' agagacaaaggacatcattt 3'
sgRNA15: upstream of the *LTF* gene promoter 5' tctgccactgtctcagcttc 3'
sgRNA16: downstream of the *LTF* gene promoter 5' cagtgctcggtgacctcacg 3'

### 2. Design of target sites of CRISPR/Cas12:

Using a Cas12 protein of which the amino acid sequence is as set forth in SEQ ID NO: 1 as an editor, target sites of CRISPR/Cas12 were respectively designed upstream and downstream of the bovine *LTF* gene, and target sites were respectively designed upstream and downstream of the 4K fragments of the promoters of the bovine *LTF, CSN1S1, CSN1S2, CSN2, CSN3, PAEP* and *LALBA.* The full-length CrRNA for the target sites were synthesized by GenScript (Nanjing) Co., Ltd., of which the sequences are as follows:
CrRNA1: upstream of the *LTF* gene 5' tccagtgctcggtgacctcac 3'
CrRNA2: downstream of the *LTF* gene 5' tgaggaattagtggtgtttga 3'
CrRNA3: upstream of the *CSN1S1* gene promoter 5' ggtcatagttgaattgtccattc 3'
CrRNA4: downstream of the *CSN1S1* gene promoter 5' ttacatagatcttgacaaccatg 3'
CrRNA5: upstream of the *CSN1S2* gene promoter 5' tgggaaaaccattaggagcttag 3'
CrRNA6: downstream of the *CSN1S2* gene promoter 5' ctttgtcctgtgagatagacaag 3'
CrRNA7: upstream of the *CSN2* gene promoter 5' caggcaagagtactggagtgggg 3'
CrRNA8: downstream of the *CSN2* gene promoter 5' atggcatgctaattttgtggttt 3'
CrRNA9: upstream of the *CSN3* gene promoter 5' agtctatggcatttgtgaattaa 3'
CrRNA10: downstream of the *CSN3* gene promoter 5' atttaattttaggtgcaatgatg 3'
CrRNA11: upstream of the *PAEP* gene promoter 5' cagattcaatgcaatccttatca 3'
CrRNA12: downstream of the *PAEP* gene promoter 5' atggctgcagctggggtcacgct 3'
CrRNA13: upstream of the *LALBA* gene promoter 5' gaactcagcattcagatgctcat 3'
CrRNA14: downstream of the *LALBA* gene promoter 5' gttaccccccaagattctgaaat 3'
CrRNA15: upstream of the *LTF* gene promoter 5' taccatagccagcttcattacgt 3'
CrRNA16: downstream of the *LTF* gene promoter 5' tccagtgctcggtgacctcacga 3'

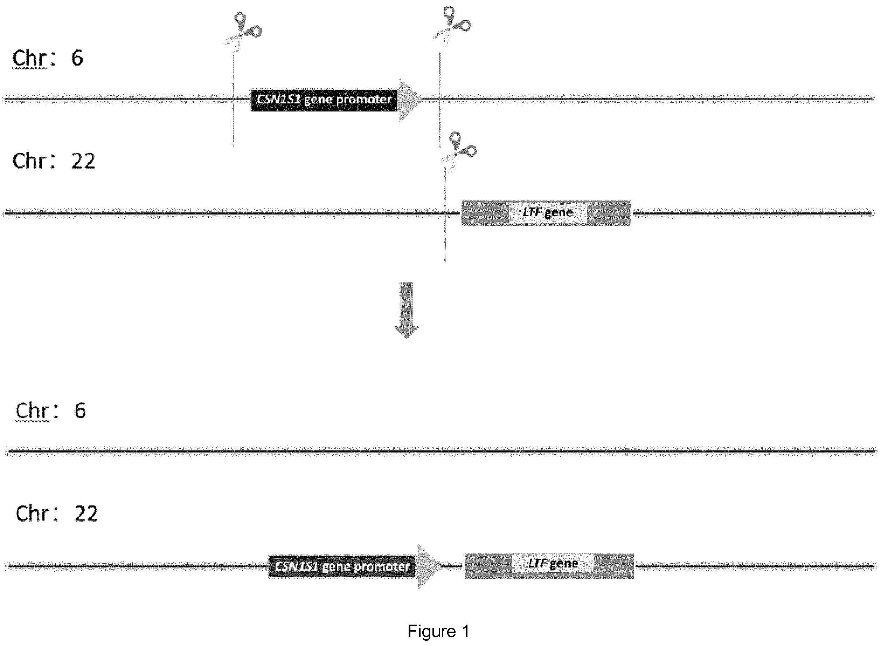

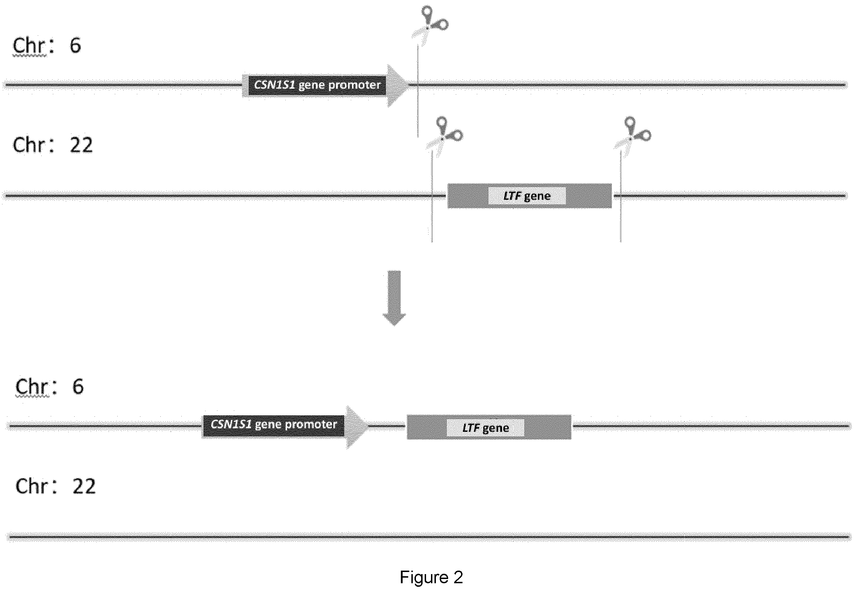

Taking the *CSN1S1* gene promoter as an example, as shown in Figure 1, under the cooperation of Cas9 and 3 sgRNA or the cooperation of Cas12 and 3 CrRNA, double-strand breaks (DSB) were formed at 3 positions, two of which were located on both sides of the promoter of a gene such as the bovine *CSN1S1,* with the purpose of cutting it off, and the third cut (DSB) was located upstream of the bovine *LTF* gene. Finally, an insertional translocation event was caused in which the promoter of a gene such as the bovine *CSN1S1* was inserted upstream of the *LTF* gene. As shown in Figure 2, under the cooperation of Cas9 and 3 sgRNA or the cooperation of Cas12 and 3 CrRNA, double-strand breaks (DSB) were formed at 3 positions, two of which were located on both sides of the bovine *LTF* gene, with the purpose of cutting it off, and the third cut (DSB) was located downstream of the promoter of a gene such as the bovine *CSN1S1.* Finally, an insertional translocation event was caused in which the *LTF* gene was inserted downstream of the *CSN1S1* gene promoter, wherein the sequence maps of the junctions between the *CSN1S1, CSN2* promoters and the *LTF* gene were shown in Figure 9.

### Example 5 Gene knock-up of lactotransferrin gene expression in mammary epithelial cells with the aid of the 4K promoters of the CSN1S1 and other genes

### 1. RNP transformation of dairy cattle mammary epithelial cells during lactation

In order to reduce the probability of integration of exogenous DNA into the dairy cattle genome, a ribonucleoprotein (RNP) complex was adopted to transfect the RNA mentioned in Example 4, i.e., the target sites upstream and downstream of the 4K fragments of the promoters of the bovine *LTF, CSN1S1, CSN1S2, CSN2, CSN3, PAEP* and *LALBA,* and the target sites upstream and downstream of the bovine *LTF* gene. Similarly, a similar gene editing event may also be obtained by means of mRNA-mediated transient expression or vector-mediated transient or stable expression.

The combinations of sgRNA 1+3+4, sgRNA 1+5+6, sgRNA 1+7+8, sgRNA 1+9+10, sgRNA 1+11+12, sgRNA 1+13+14 and sgRNA 1+15+16 were employed in the CRISPR/Cas9 system to transfect dairy cattle mammary epithelial cells; the combinations of CrRNA 1+3+4, CrRNA 1+5+6, CrRNA 1+7+8, CrRNA 1+9+10, CrRNA 1+11+12, CrRNA 1+13+14 and CrRNA 1+15+16 were employed in the CRISPR/Cas12 system to transfect dairy cattle mammary epithelial cells. The cells with the correctly edited genotypes were identified by PCR 48 hours after transfection and then the single clones were picked. Eventually the single cloned cell lines with the correctly edited genotypes were obtained for subsequent assays.

### 2. Identification of lactotransferrin expression knocked up by gene editing in mammary epithelial cells

The various single cloned cell lines with correctly edited genotypes were respectively induced by prolactin, and the expression levels of the lactotransferrin gene of the cell lines were detected and the lactotransferrin content was determined.

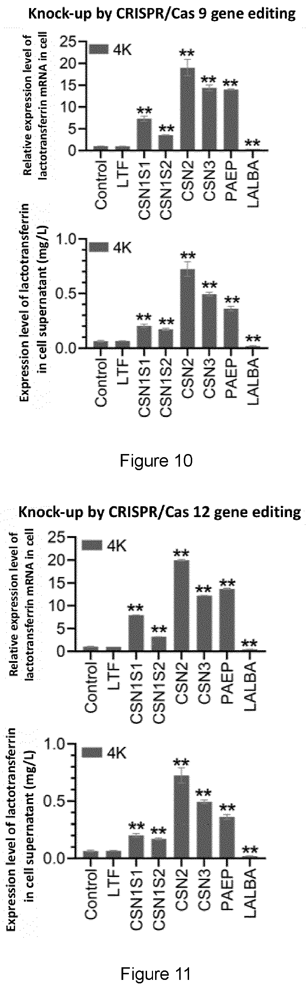

The knock-up results of both CRISPR/Cas9 and CRISPR/Cas12 (SEQ ID NO: 1) gene editing showed that, there was no significant difference in the lactotransferrin expression levels between the unedited control cells (Control) and the cells knocked up using the *LTF*'*s* own promoter; the knock-up using the *CSN2* promoter fragment significantly increased the expression of mRNA in the cells and lactotransferrin expression in the cell supernatant; the knock-up using the *CSN3, PAEP* and *CSN1S1* promoter fragments also distinctly increased lactotransferrin expression levels; the knock-up using the *CSN1S2* promoter also led to a certain increase; while the *LALBA* promoter, on the contrary, reduced the expression level of lactotransferrin (Figure 10 and 11).

The above results observed from the cells demonstrated that, knocking up lactotransferrin by gene editing with the bovine *CSN2, CSN3, PAEP, CSN1S1* and *CSN1S2* promoters can effectively increase the expression of lactotransferrin.

### Example 6 Establishment of bovine somatic fetal fibroblast cell lines required for gene-edited cattle

1. Collection of fetal fibroblasts of Holstein dairy cattle (cows and bulls). A 45-day Holstein dairy cattle fetus was immersed and disinfected in 75% ethanol, and then stripped of the fetal membrane, and washed twice in a PBS solution added with penicillin-streptomycin. Except for the head, limbs and internal organs, the tissue samples were cut into pieces, digested with trypsin, and cultured in a DMEM/F12 + 10% FBS medium at 38.5°C in an incubator with 5% CO₂. The next day, the adhesion state and growth state of the cells were observed under a microscope, and the medium was replaced with a fresh one. The cells were digested with trypsin after the cells were confluent and cryopreserved in liquid nitrogen.
2. Cell thawing and passage. After taken out of the liquid nitrogen, the frozen cells were quickly thawed in a water bath at 37°C. The cells were resuspended after added with serum-free medium and centrifuged, inoculated on a cell petri dish with medium, and cultured at 38.5°C in an incubator with 5% CO₂. When the cells grew to about 70% confluence, the culture was enlarged by passage. Dead cells and culture medium were washed off with PBS. A small amount of trypsin was added to digest the cells, and after 30-60 s, the trypsin was sucked out. The cells were cultured and pipetted to become a cell suspension which was divided into each petri dish at a ratio of 1:2 or 1:3.

### Example 7 Transfection of fibroblasts and selection of single clones

1. Fetal fibroblasts of dairy cattle were thawed, passaged for one generation, and transfected with RNP when the confluence between the cells was 70-80%.
2. The RNP complexes of sgRNA-Cas9 and CrRNA-Cas12 types were respectively added to diluted Lipofectamine^{®} RNAiMax, mixed well, and incubated at room temperature for 20 min to form transfection complexes which were eventually added to the cells. The media were replaced with fresh ones 12 hours after transfection.
3. After 48-72 h, the cells with the correct genotypes were identified and the single clones were then spread when the cells had covered the 24-well plate.
4. When the clones basically covered a field of view under a microscope, single clones were picked using a cloning cylinder and cultured.
5. Identification. The genomic DNA of the cells were extracted for PCR identification.

### Example 8 Preparation of somatic cell cloned cattle

### 1. Preparation of donor cells

Fibroblasts that had been successfully edited after transfection and had normal karyotypes were used as donor cells for nuclear transfer. The cells were grown to contact inhibition, then added with 0.5% FBS, and starved for 2 to 7 days or contact-inhibited for 3 days or more to induce the cells to enter the G0 phase. Three hours before nuclear transfer, culture medium was discarded. The cells were washed 3 times with DPBS, added with 0.25% trypsin to digest for 2-3 min at 37°C, then added with 2 mL of culture medium to terminate the digestion, and repeatedly pipetted with a micropipette to obtain scattered single cells.

### 2. Maturation culture and enucleation of oocytes

After maturation culture in vitro, the bovine oocytes were transferred to a tube of 0.1% hyaluronidase, shaken for 2-3 min, and pipetted with a micropipette to completely separate the cumulus cells from the oocytes. The oocytes were collected and washed three time with maturation culture medium. The oocytes with homogeneous cytoplast, integral morphology and first polar bodies were selected as the somatic cell nuclear receptors.

They were then transferred to a working solution of M199 + 10% FBS + 7.5 µg/mL cytochalasin B, and the chromosomes in the first polar bodies and oocytes were removed with a glass needle under a microscope. They were washed 3 times with M199 + 20% FBS solution and placed at 38.5°C in an incubator with 5% CO₂.

### 3. Nuclear transfer and fusion

The donor cells were treated by serum starvation for 2-4 days, and digested with 0.25% trypsin for 5 min. Somatic cells with a diameter of 10-12 µm were selected and transferred to the zona pellucida of the enucleated oocytes, and then transferred to the fusion chamber after equilibrated in Zimmerman solution for 5 min, so that the contact surface between the donor cells and the oocytes were perpendicular to the electric field. Fusion was carried out under the conditions of DC pulse field strength of 2.5 kV/cm, pulse duration of 10 µs, pulse frequency of 2, and pulse interval of 1 s. The reconstructed embryos were quickly transferred to M199 + 10% FBS solution and placed for 0.5 h to then observe the fusion.

### 4. Activation and culture of reconstructed embryos

Well-fused reconstructed embryos were transferred to 5 µM ionomycin solution for 4-min treatment, and then transferred to 1.9 mM 6-DMAP solution for 4-h treatment. Subsequently, the reconstructed embryos were transferred to CR1aa + 5% FBS solution and cultured at 38.5°C in an incubator with 5% CO₂ for 2 days to observe cleavage and count the cleavage rate. After 7 days, the development of blastocysts was observed and the development rate was counted.

### 5. Embryo transplantation and pregnancy test

The day 7 cloned blastocysts with good morphology were transplanted into the uterine horns of the synchronized recipient cows. A rectal examination was performed to determine the pregnancy rate on day 60 after the transplantation.

### Example 9 Lactogenesis of the dairy cattle offspring of gene knock-up cows and gene knock-up bulls and cow milk composition identification

### 1. Induction of lactation

The cows of 6-8 months old were subcutaneously injected with 25 mg/kg/d medroxyprogesterone acetate and 7.5 mg/kg/d estradiol benzoate for 7 consecutive days to induce lactation, resting for 2 days, followed by intramuscular injection of 4 mg/d dexamethasone for 3 days. The cows were milked from the day after dexamethasone injection, twice a day. After stopping milking, the cows were intramuscularly injected with 30 mg/kg spiramycin for 3 days in a row to prevent mastitis.

### 2. Identification of cow milk composition

The collected milk sample was mixed evenly, filtered through gauze, put into the sampling tube for dairy cow production performance measurement, stored at 4°C, and then transported to the dairy cow production performance measurement center for determination of milk composition, especially the lactotransferrin content. Through the effect of the above related promoters on increasing the bovine lactotransferrin expression verified by the present invention, it can be inferred that the bovine lactotransferrin content in this experiment was significantly increased.

All publications and patent applications mentioned in the specification are hereby incorporated herein by reference, just as each publication or patent application is separately or particularly incorporated by reference into herein.

Although the foregoing invention has been described in detail through illustrations and examples for clear understanding, obviously some changes and modifications can be implemented within the scope of claims attached. Such changes and modifications are within the scope of the present invention.

## Claims

1. A method of specifically increasing *LTF* gene expression levels in mammary glands, wherein it is **characterized in that** it adopts the promoter of a specific gene, which is selected from αS1 casein gene, β casein, αS2 casein, κ casein, β-lactoglobulin or α-lactalbumin genes, to drive the *LTF* gene expression; preferably, it comprises the following steps: DNA breaks are generated simultaneously at three different specific sites in the genome of an animal cell, wherein the specific sites are respectively two genomic loci where the promoter fragment of the specific gene can be cut out and a genomic locus between the coding region of the *LTF* gene and the promoter; or a genomic locus between the promoter of the specific gene and the coding region, and two genomic loci where the coding region fragment of the *LTF* gene can be cut out; a translocation editing event, in which the promoter fragment of the specific gene is inserted upstream of the coding region of the *LTF* gene or the coding region fragment of the *LTF* gene is inserted downstream of the promoter of the specific gene, is then generated by gene editing at the above loci; eventually, a new combination of the promoter of the specific gene and the coding region of the *LTF* gene is produced, that is, the promoter of the specific gene is employed to drive the *LTF* gene expression, thereby increasing the *LTF* gene expression level in mammary glands.

2. A method of creating a new gene in animal cells, wherein it is **characterized in that** it comprises the following steps:
(1) DNA breaks are generated simultaneously at three different specific sites in the genome of an animal cell, wherein the specific sites are respectively two genomic loci where the promoter fragment of the specific gene can be cut out and a genomic locus between the coding region of the *LTF* gene and the promoter; or a genomic locus between the promoter of the specific gene and the coding region, and two genomic loci where the coding region fragment of the *LTF* gene can be cut out; a translocation editing event, in which the promoter fragment of the specific gene is inserted upstream of the coding region of the *LTF* gene or the coding region fragment of the *LTF* gene is inserted downstream of the promoter of the specific gene, is then generated by gene editing at the above loci; eventually, a new combination of the promoter of the specific gene and the coding region of the *LTF* gene is produced to form a new gene; the specific gene is selected from αS1 casein gene, β casein, αS2 casein, κ casein, β-lactoglobulin or α-lactalbumin genes;
preferably, further comprising (2) designing primer pairs capable of specifically distinguishing the above new combination, screening out cells or tissues containing the above new gene by PCR identification, and determining the characteristic sequence of the new combination by sequencing; and
(3) cultivating the above screened cells or tissues to obtain TO-generation animals, and performing PCR identification and sequencing on animals of two consecutive generations of T0 and T1, or three or more consecutive generations to screen out animals that stably inherit the characteristic sequence comprising the above new combination over different generations, that is, creating a new gene that is stably inheritable in the animals;
optionally, also comprising (4) testing the functionality-related biological traits or phenotypes of the new gene, determining a genotype that can bring beneficial traits, and acquiring a new functional gene that is stably inheritable.

3. The method according to claim 1 or 2, wherein it is **characterized in that**, the "three different specific sites" are located on the same chromosome or on different chromosomes; preferably, at specific sites on three different genes, or at three different specific sites on the same gene, and the transcription directions of the "three different genes" may be the same or different.

4. The method according to any one of claims 1 to 3, wherein it is **characterized in that**, the "DNA break" is achieved by delivering a nuclease with targeting properties into a cell of an organism to contact with specific sites of the genomic DNA; preferably, the "nuclease with targeting properties" is meganuclease, zinc finger nuclease, TALEN or CRISPR/Cas systems (e.g., Cas9 nuclease system or Cas12 nuclease system); more preferably, the "nuclease with targeting properties" exists in the form of DNA, or in the form of mRNA or a protein instead of DNA.

5. The method according to claim 4, wherein it is **characterized in that**, the method of delivering a nuclease with targeting properties into a cell is selected from 1) PEG-mediated cell transfection method; 2) liposome-mediated cell transfection method; 3) electroporation transformation method; 4) microinjection; 5) particle bombardment; 6) *Agrobacterium-*mediated transformation method; 7) viral vector-mediated transformation method; or 8) nano-magnetic bead transformation method.

6. A new gene produced by the method according to any one of claims 2 to 5.

7. An application of the gene according to claim 6 in increasing the lactotransferrin content in animal milk (preferably cow milk).

8. A DNA comprising the gene according to claim 6.

9. A protein encoded using the gene according to claim 6, or a bioactive fragment thereof.

10. A recombinant expression vector comprising the gene according to claim 6 and a promoter operably linked thereto.

11. An expression cassette comprising the gene according to claim 6.

12. An animal cell or an animal regenerated therefrom, wherein it comprises the expression cassette according to claim 11.

13. A composition, comprising the promoter of the specific gene and the coding region of the *LTF* gene, wherein, the specific gene is selected from αS1 casein gene, β casein, αS2 casein, κ casein, β-lactoglobulin or α-lactalbumin genes; the composition is non-naturally occurring and is directly linked on animal chromosomes and can be stably inherited; preferably, it fuses in vivo.
